# DEMANDE DE BREVET EUROPEEN

(11) **EP 1 359 421 A1**
(43) Date de publication de la demande: **05.11.2003**
(21) Numéro de dépôt: 03101200.8
(22) Date de dépôt: 30.04.2003
(51) Int. Cl.: G01N 33/533

(54) **Procédé de détection d'une reconnaissance moléculaire par électrochimiluminescence**

(30) Priorité: 03.05.2002 FR 0205562
(71) Demandeur: COMMISSARIAT A L'ENERGIE ATOMIQUE, 75752 Paris Cédex 15 (FR); UNIVERSITE JOSEPH FOURIER, 38041 Grenoble Cédex 9 (FR); UNIVERSITE CLAUDE BERNARD - LYON 1, F-69622 Villeurbanne Cédex (FR)
(72) Inventeur: Billon, Martial, 38100 Grenoble (FR); Bidan, Gérard, 38100 Grenoble (FR); Dupont Filliard, Agnès, 38000 Grenoble (FR); Blum, Loic, 69300 Caluire (FR)
(74) Mandataire: Poulin, Gérard

(57) **Abrégé**

La présente invention se rapporte à un procédé de détection d'une reconnaissance moléculaire par électrochimiluminescence. Ce procédé comprend les étapes consistant a) à déposer sur un support un film conducteur électronique et à fixer sur ledit film une molécule sonde pour obtenir un support test ; b) à soumettre l'échantillon à tester à un protocole de marquage de la molécule cible avec un marqueur électrochimiluminescent ; c) à mettre en contact la cible marquée avec le support test ; d) à rincer le support test ; et e) à soumettre le support test rincé de l'étape d) à une lecture du signal d'électrochimiluminescence déclenchée par un transfert électronique direct, ou indirect, entre la cible et le support via le film conducteur électronique.

## Description

### Domaine technique

La présente invention se rapporte à un procédé de détection d'une reconnaissance moléculaire par électrochimiluminescence. Elle concerne de manière générale la détection qualitative et quantitative d'une reconnaissance moléculaire spécifique entre une première molécule fixée sur un support et une deuxième molécule recherchée dans un échantillon.

Selon l'invention, la reconnaissance moléculaire spécifique peut être définie comme une interaction spécifique entre deux molécules plus ou moins complexes, conduisant à une liaison ou assemblage des deux molécules suffisamment stable pour que les molécules puissent être détectées lorsqu'elles sont liées. Dans la présente invention, il peut s'agir par exemple d'une hybridation d'acides nucléiques (ADN et/ou ARN), d'une réaction de reconnaissance de type antigène/anticorps, d'une interaction de type protéine/protéine, d'une interaction de type enzyme/substrat, etc.

Le procédé de la présente invention trouve par exemple une application dans la détection dans le domaine des "Biopuces" au sens large c'est-à-dire des puces à acides nucléiques et des puces à protéines ou tous systèmes y compris multiparamétriques pour l'étude de la reconnaissance sonde-cible de substrats biologiques. Il s'agit par exemple de la détection d'hybridation d'acides nucléiques sur support solide, en milieu aqueux ou à l'air, par exemple dans le cadre d'un criblage ("screening") ou d'une détection d'hybridation sur une biopuce.

Le procédé de la présente invention peut être appliqué à toutes finalités de marquage de surfaces conductrices par un système électrochimiluminescent.

### Etat de la technique

Les biocapteurs miniaturisés ou biopuces sont actuellement l'objet d'enjeux économiques majeurs. Cet attrait s'explique par leur capacité de pouvoir réaliser des milliers d'analyses en parallèle ainsi que par leur vaste champ d'application touchant à la fois le domaine du diagnostic médical, du contrôle environnemental et agro-alimentaire, ainsi que celui de la pharmacologie.

Ces systèmes sont constitués de molécules sondes, généralement de molécules biologiques telles que des fragments d'ADN, ou plus généralement des fragments d'oligonucléotides (ou ODN), immobilisées sur de très petites surfaces. Ces sondes, de par leur capacité de reconnaître spécifiquement des entités biologiques données, appelées molécules cibles, comme des brins d'ADN ou d'ODN complémentaires, confèrent au biocapteur une sélectivité de reconnaissance.

La sensibilité de ces biocapteurs dépend en partie de la technique mise en oeuvre pour révéler le phénomène de reconnaissance molécules sondes/molécules cibles. De nombreux outils ont été développés et plus particulièrement certains utilisant des marqueurs fluorescents qui offrent une grande sensibilité de détection.

Cependant, ces techniques nécessitent une excitation laser des fluorophores greffés sur les biomolécules cibles qui peut s'accompagner également par une fluorescence résiduelle parasite provenant de l'illumination du support et des biomolécules.

Dans la présente description, les références entre crochets [] renvoient à la liste de références annexée.

L'électrochimiluminescence (ECL) ou chimiluminescence électrogénérée est un phénomène basé sur l'émission de lumière via une réaction électrochimique. L'ECL a été employée comme moyen de détection [1] et [2]. Parmi les marqueurs électrochimiluminescents les plus communément employés, on distingue les composés types organométalliques tel que le complexe Ru(2,2'-bipyridine)₃²⁺ et les composés organiques comme le luminol et dérivés.

Bien que désignés dans les publications et revues [1] par la même appellation ECL, ces deux familles donnent lieu à des mécanismes d'ECL très différents en particulier les composés organométalliques ne sont pas épuisés par la réaction et l'émission ECL est continue, liée au maintien d'un potentiel électrique adéquat et à la présence d'un co-substrat ; tandis que la molécule de luminol, ou ses dérivés, est consommée irréversiblement. Nous parlerons dans le cas du luminol de chimiluminescence électrodéclenchée (ETCL : « Electro-Triggered Chemiluminescence »).

C'est Blakburn et all. [3] qui ont été les premiers à utiliser la détection par ECL dans le cas de la détection de produits issus de la réaction en chaîne de la polymérase (PCR) en greffant préalablement le complexe Ru(bpy)₃²⁺ sur des protéines et des acides nucléiques. La limite de détection a été de l'ordre du subpicomolaire avec un domaine de linéarité de plus de six ordres de grandeur. En outre, ces marqueurs montrent une très grande stabilité et peuvent être stockés plus d'une année dans le noir et à température ambiante. Enfin, il est possible de greffer plusieurs marqueurs Ru(bpy)₃²⁺ sur une même biomolécule sans en affecter sa réactivité ou ses propriétés de reconnaissance. Xu et coll. [4] ont également appliqué ce mode de détection par ECL à un biocapteur à ADN constitué de simples brins d'ADN immobilisés sur une électrode recouverte par un assemblage moléculaire d'alcane bisphosphonate d'aluminium. Le phénomène d'hybridation entre ces fragments d'ADN immobilisés et des brins complémentaires en solution a été mis en évidence via ECL de complexes Ru(bpy)₃²⁺ soit insérés comme intercalant ou greffés préalablement sur les brins complémentaires.

Le luminol a été également utilisé comme marqueur ECL dans un immunocapteur mis en oeuvre pour la détection de l'acide 2,4-dichlorophenoxyacétique (2,4-D), herbicide connu pour son caractère potentiellement cancérigène [5]. Le 2,4-D sous sa forme ester activé est préalablement ancré sur une électrode en carbone portant des chaînes aminohexane. Puis par reconnaissance de cet herbicide par un anticorps portant du luminol, il a été possible d'estimer la quantité d'herbicide immobilisée via l'intensité générée par ECL des luminophores en présence de H₂O₂. Cette immunodétection du 2,4-D par ECL a montré qu'il était possible de détecter une concentration limite équivalente à 0,2 µgl⁻¹.

Malheureusement ce procédé ne peut pas conduire à un système multiparamétrique. En effet, l'ancrage de l'analyte 2,4-D s'effectue de manière non-spécifique sur une couche auto-assemblée composée de chaînes d'aminohexane par simple couplage chimique entre une fonction amine portée par une des chaînes aminohexane et la fonction acide sous sa forme ester activée portée par une molécule 2,4-D.

Des intercalants électrochimiluminescents ont été utilisés pour détecter l'hybridation de l'ADN tel que décrit dans le document [6]. Le procédé consiste à immobiliser l'échantillon d'ADN cible sur une électrode formant le fond d'une cellule électrochimique puis la sonde ADN et la substance ECL présentant des propriétés de liaison spécifique avec un double brin sont alors ajoutées. Des intercalants chimiluminescents comme l'acridine et le lucigène ont été utilisés pour une détection par ECL. Dans ce document, le luminol qui n'est pas un intercalant de l'ADN n'a pas été utilisé dans un mécanisme ETCL, mais sa chimiluminescence déclenchée par des méthodes chimiques, le luminol étant soit en solution, la séquence sonde étant greffée par une peroxydase, soit greffé sur la séquence sonde.

Cependant, l'interaction ADN/intercalant n'est pas toujours sélective. En outre, l'intercalant interagit de manière non spécifique avec les simples brins ADN sondes et s'adsorbe sur le film ce qui induit un signal parasite. C'est pourquoi cette méthode d'immobilisation de la sonde ECL par intercalation ne permet pas une lecture en parallèle et multiparamétrique.

Les techniques de l'art antérieur ne sont donc pas toujours précises, et, souvent, ne permettent pas d'être mises en oeuvre sur un système multiparamétrique (« multi-array ») ou sur un système du type de la puce MICAM commercialisée par la société CisBio (marque déposée).

### Exposé de l'invention

La présente invention a précisément pour but de résoudre les problèmes précités de l'art antérieur en fournissant un procédé de détection d'une reconnaissance moléculaire entre une molécule sonde fixée sur un support et une molécule cible recherchée dans un échantillon à tester, ledit procédé permettant en outre une détection quantitative et qualitative précise et sensible de la molécule cible lorsqu'elle est présente dans l'échantillon.

Le procédé de la présente invention comprend les étapes suivantes :
a) déposer sur un support un film conducteur électronique à conduction électronique ou rédox et fixer sur ledit film une molécule sonde pour obtenir un support test,
b) marquer avant ou après l'étape c) la molécule cible avec un marqueur électrochimiluminescent,
c) mettre en contact la molécule cible marquée ou non marquée avec le support test dans des conditions physiques et chimiques permettant la reconnaissance moléculaire et l'assemblage spécifique entre la molécule sonde et la molécule cible,
d) rincer le support test de manière à supprimer l'excès de marqueur électrochimiluminescent tout en préservant l'assemblage spécifique entre la molécule sonde et la molécule cible de l'étape c), et
e) soumettre le support test rincé obtenu à l'étape d) à une lecture d'électrochimiluminescence déclenchée par un transfert électronique direct, ou indirect, entre la cible et le support via le film conducteur électronique.

La détection d'une chimiluminescence à l'étape e) révèle un assemblage par reconnaissance moléculaire au sens précité entre la molécule sonde fixée sur le support et la molécule cible recherchée, et donc la présence de la molécule cible dans l'échantillon testé.

Dans le procédé de la présente invention, la détection de la reconnaissance entre la molécule sonde et la molécule cible marquée est basée sur la chimiluminescence électrogénérée, ou électrochimiluminescence (ECL). Il s'agit d'une détection optique permettant de s'affranchir des inconvénients des procédés de l'art antérieur tout en conservant la sensibilité des détecteurs optiques. En effet, le déclenchement électrique de la chimiluminescence ne concerne que le marqueur chimiluminescent et non pas le support test ou les biomolécules. En outre, ce nouveau mode de détection appliqué aux biopuces permet d'effectuer des mesures quantitatives de la reconnaissance molécules sondes/molécules cibles contrairement à la détection par fluorescence. Enfin, l'excitation par une impulsion électrique conforme à la présente invention permet un contrôle spatial et une mise en oeuvre rapide et aisée tout en s'affranchissant d'un appareillage coûteux d'excitation laser utilisé dans l'art antérieur.

La présente invention trouve une application dans le domaine des biopuces, où le support sur lequel est déposé le film conducteur conformément à la présente invention forme la biopuce.

Selon l'invention, le support peut être un quelconque des supports utilisés par l'homme du métier pour la fabrication de biopuces. A titre d'exemples non limitatifs, peuvent être utilisés un support en métal tel que Au, Pt, etc., un support verre/ITO, un support en verre ou quartz métallisé, un support plastique/ITO, un support en plastique métallisé, un support en carbone vitreux, ou un support formé par dépôt d'un matériau conducteur sérigraphié sur un substrat isolant ou sur un des supports précités.

Selon la présente invention, le film conducteur peut être un film conducteur électronique intrinsèque ou rédox.

Lorsqu'il s'agit d'un film conducteur électronique, il peut s'agir d'un film de polymère conducteur, par exemple tel que ceux utilisés par l'homme du métier pour la fabrication de biopuces sur lesquelles sont greffées des sondes. Par exemple le polymère peut être un polymère conducteur tel que ceux décrits dans les « Techniques de l'Ingénieur » -A3140-sous la dénomination « polymères conducteurs intrinsèques» : il s'agit de polymères formés de molécules porteuses de liaisons conjuguées et éventuellement dopés par des dopants donneurs ou accepteurs d'électrons tels que les poly(acétylène), le poly(nitrure de soufre), le polyphénylène, le polypyrrole, le poly(sulfure de phénylène), le polythiophène, le polyaniline etc.

Selon la présente invention, le film polymère conducteur peut être déposé sur le support en utilisant les techniques classiques connues de l'homme du métier, par exemple l'électrodéposition ou encore l'électropolymérisation.

Selon l'invention, le film conducteur peut être élaboré à partir de simples monomères type pyrrole ou d'oligomères pré-synthétisés comme des oligothiophènes, ainsi qu'à partir de systèmes moléculaires plus complexes comme des unités complexantes de métaux de transition tels que les phénantrolines, la phénylpyridine, etc., portant des unités électropolymérisables ce qui conduit lors de l'élaboration du polymère à un réseau conjugué.

Selon la présente invention, lorsque le film conducteur électronique est de type rédox, ce peut être un polymère rédox constitué par une matrice polymère de type poly(vinylimidazole) renfermant des centres rédox comme un métal de transition tel que l'osmium, le ruthénium, etc. complexé par des bipyridine. Un exemple d'un poly(4-vinylpyridine) renfermant un complexe de ruthénium ayant des propriétés d'électrochimiluminescence utilisable dans la présente invention est décrit dans [8].

Selon l'invention, la molécule sonde peut être par exemple de l'ADN, un oligonucléotide, un anticorps, une protéine ou un enzyme, ainsi que toute molécule ou biomolécule permettant une reconnaissance spécifique et un assemblage tels que définis ci-dessus.

Selon l'invention, la fixation de la molécule sonde sur le film conducteur, lorsque celui-ci est un polymère conducteur peut se faire par les méthodes classiques de chimie utilisées pour fixer des sondes sur une biopuce. La liaison entre le support et la sonde peut être d'adsorption, électrostatique, chimique obtenue par auto-assemblage, silanisation, ou par toute méthode chimique, électrochimique, photochimique, etc. connue de l'homme du métier pour le dépôt de la molécule sonde par exemple fonctionnalisée par un groupement apportant la propriété d'auto-assemblage, d'ancrage, de couplage, ou polymérisable chimiquement, électrochimiquement, photochimiquement ou électrophotochimiquement par photosensibilisation.

Selon l'invention, liaison entre le film déposé sur le support et la sonde peut être obtenue en une étape par exemple par le procédé MICAM (marque déposée).

Les documents FR-A-2 787 581, FR-A-2 787 582 et US 5 810 989 décrivent par exemple des techniques de dépôt de film et de fixation de molécules sondes utilisables dans la présente invention. Il s'agit par exemple d'électro-copolymérisation sur un support, par exemple sur un substrat de silice, de monomères précurseurs du polymère conducteur, tel que le pyrrole, avec des monomères, par exemple de pyrrole, fonctionnalisés par une molécule sonde selon la présente invention, par exemple des oligonucléotides. Dans ces techniques, on exploite l'adhésion du film conducteur de polypyrrole sur le substrat de façon à y réaliser la fixation des molécules sondes de reconnaissance.

Selon l'invention, la sonde peut aussi être fixée sur le film déposé sur le support par exemple par post-fonctionnalisation du film, par exemple de polypyrrole (Ppy), par exemple déposé par électrogreffage sur le support, par un système de reconnaissance par affinité, par exemple un système avidine/biotine ou des systèmes équivalents, ou des dérivés de ces systèmes.

Ceci conduit par exemple aux structures film conducteur-molécule sonde suivantes : PPy-ADN ; PPy-biotine/avidine/biotine-ADN ; PPy--biotine/avidine-protéine ; PPy-biotine/avidine/biotine-anticorps, utilisables selon la présente invention. Ces structures sont par exemple utilisables sur un support d'or (Au) ou de silice, ou sur tout support permettant le greffage du polymère conducteur.

Cette fixation peut se faire par exemple avec adressage des sondes. Il s'agit par exemple de l'adressage photochimique, de l'adressage mécanique, par exemple par micropipetage à l'aide d'un robot disperseur, et de l'adressage électrochimique. Ces techniques de fixation de sondes sur un film polymère sont connues de l'homme du métier. L'adressage permet des analyses multiparamétriques.

L'ancrage de la molécule sonde peut se faire par simple couplage entre deux fonctions chimiques réactives, par exemple type ester activé et une amine, l'une de ces fonctions étant portée par la molécule sonde et l'autre ancrée sur le film. Le couplage chimique sonde/film peut être empêché en bloquant la réactivité de la fonction chimique portée par le film. L'empêchement peut être levé, « débloqué », en rendant à nouveau la fonction réactive, ceci grâce par exemple à une activation électrochimique ou photochimique. A l'issue, le couplage de la molécule sonde sur le film est à nouveau possible.

Selon l'invention, le marqueur peut être un des marqueurs électrochimiluminescents connus de l'homme du métier. Par exemple, il peut être choisi dans le groupe constitué du luminol, de l'isoluminol, d'un aminophtalhydrazine et de leurs dérivés.

Selon l'invention, on entend par dérivé du luminol ou de l'isoluminol les composés des dérivés des formules suivantes : 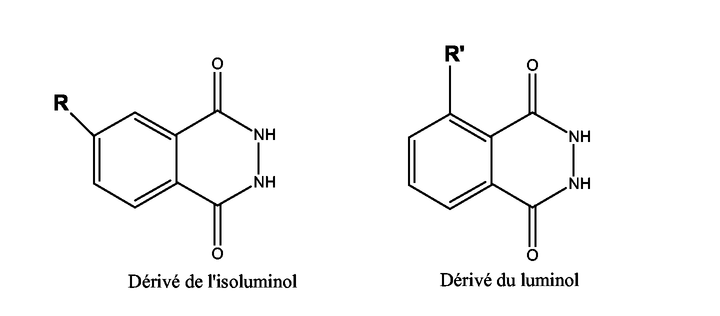 dans lesquelles R ou R' sont des groupements actifs qui permettent une fonctionnalisation, c'est à dire une modification chimique permettant la fixation du marqueur sur la molécule cible conformément à la présente invention. A titre d'exemples non limitatifs, on peut citer R ou R' = H₂N-(H₂C)₃-NH ; NH₂-(CH₂)₄-(C₂H₅)N ; chaînes alkyles ou alkoxy substituées ou non et leur combinaisons ou dérivés. Ces marqueurs sont disponibles dans le commerce, par exemple le N-(4-aminobutyl)-N-éthylisoluminol sous la marque ABEI fabriqué par la société SIGMA.

Selon l'invention, la liaison entre la molécule cible et le luminol, s'effectue au travers du substituant R ou R'. Celui-ci est donc choisi en fonction de la réactivité de la molécule cible.

La liaison peut être directe, par exemple ADN-luminol ou protéine-luminol, ou indirecte par le biais d'un couplage par affinité de type biotine/avidine, par exemple ADN_{cible}-biotine/avidine-luminol.

Des protocoles de marquage utilisables dans la présente invention sont décrits par exemple dans les documents [9] et [10].

Selon l'invention, le marquage de la cible par le luminol peut être antérieur ou postérieur à l'étape c) de mise en contact de la molécule cible avec le support test. En d'autres termes, le marquage de la molécule cible peut être effectué soit sur l'échantillon à tester avant sa mise en contact avec le support test, c'est à dire avant que l'assemblage molécule sonde/molécule cible se fasse, soit sur le support test après l'étape c) de mise en contact de la molécule cible avec le support test, c'est à dire, après que l'assemblage molécule sonde/molécule cible se soit fait. L'homme du métier saura adapter le procédé de la présente invention par exemple suivant le type de molécules sonde/cible mises en jeu.

L'étape c) de mise en contact de la molécule cible avec le support test est bien entendu réalisée dans des conditions physiques et chimiques permettant la reconnaissance moléculaire et l'assemblage spécifique entre la molécule sonde et la molécule cible. Ces conditions sont par exemple celles qui permettent l'hybridation de brins d'acides nucléiques complémentaires dans le cas de molécules sondes et cibles d'acides nucléiques, ou celles qui permettent une reconnaissance et une interaction de type protéine/protéine dans le cas de molécules sondes/cibles de type protéique. Elles sont connues de l'homme du métier.

La reconnaissance entre molécule cible et molécule sonde lors de l'étape c) de mise en contact conduit à un assemblage spécifique moléculaire sonde /molécule cible : il s'agit d'une association supramoléculaire ou suprabiomoléculaire résultant d'une reconnaissance ou affinité entre une sonde immobilisée sur le support et un analyte cible en solution. Des exemples ont été cités ci-dessus.

L'étape d) de rinçage consiste à retirer l'excès de marqueur sur le support, c'est à dire les molécules de marqueur non liées à des molécules cibles. Elle peut être réalisée par exemple au moyen d'eau physiologique ou de toute solution préservant l'assemblage molécule sonde/molécule cible sur le support.

L'étape e) est une lecture d'électrochimiluminescence. Selon l'invention, cette luminescence est déclenchée par un transfert électronique entre le marqueur de la molécule cible et le support conducteur électrique directement, ou indirectement, via le film conducteur électronique.

Dans cette étape, le support sur lequel est déposé le film conducteur sert d'électrode pour faire passer un courant électrique en appliquant un potentiel au support de manière à déclencher l'électrochimiluminescence. Le film conducteur est bien entendu mis dans un état de conductivité qui permet ou favorise le transfert d'électrons précité.

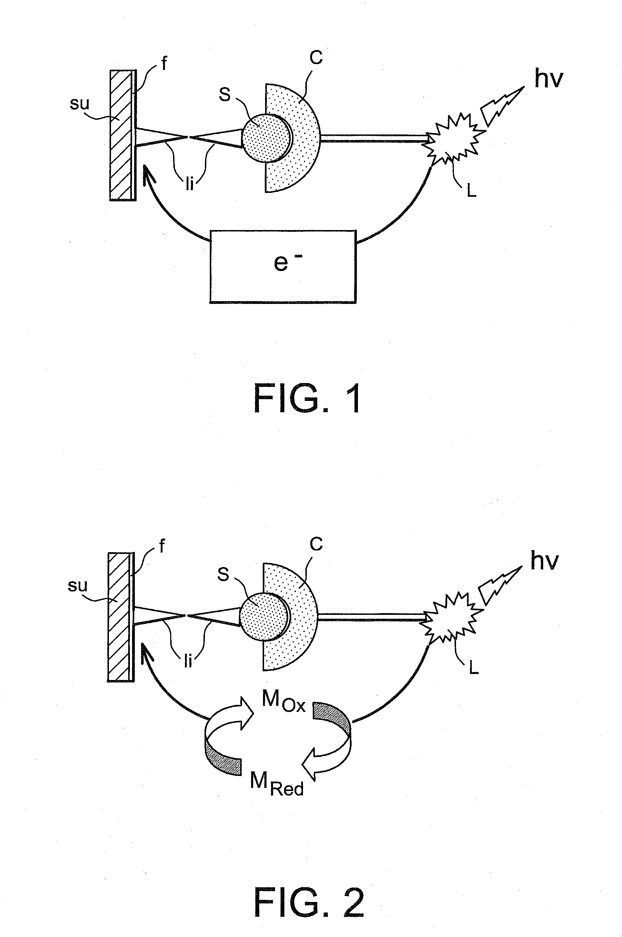

Les figures 1 et 2 sont des représentations schématiques d'un transfert électronique direct (figure 1) ou indirecte (figure 2) entre la molécule cible marquée et le support via le film conducteur électronique lors d'une lecture d'électrochimiluminescence conformément au procédé de la présente invention. Sur ces figures, « su » représente le support, « f » le film conducteur électronique, « li » la liaison film conducteur électronique/molécule sonde, « S » la molécule sonde, « C » la molécule cible, « L » le luminol, « e⁻ » le transfert électronique directe (figure 1) et « Mox/Mréd » un couple rédox appelé médiateur (M) rédox (ox = oxydant ; réd = réducteur) permettant un transfert électronique indirecte par son intermédiaire.

Selon l'invention, le médiateur redox entre le support et le luminol a pour but de transférer les électrons du luminol vers le support. Il peut être en solution, ou co-immobilisé sur le support avec le l'assemblage ou relié à l'assemblage. A titre d'exemples non limitatifs, il peut être choisi parmi : un groupement métallocène tel que le ferrocène ou le diferrocène, des complexes de métaux de transition tels qu'un complexe de cobalt, en solution ou greffé au film conducteur, par exemple de polypyrrole, ou à la sonde, ou des intercalants de l'ADN possédant des propriétés redox ou comportant des groupements redox. L'homme du métier saura adapter le procédé de la présente invention en fonction des molécules cibles et sondes en jeu.

L'étape finale de lecture d'électrochimiluminescence peut être réalisée au moyen d'un luminomètre qui mesure l'intensité de luminescence émise. L'intensité de luminescence émise est proportionnelle à la concentration de molécules cibles assemblées aux molécules sondes.

Dans le procédé de la présente invention, le transfert électronique du support vers le luminol s'effectue au travers d'un film conducteur électronique ce qui n'est pas le cas dans les systèmes développés par Xu et coll. [4] et Marquette et coll. [5]. En effet pour ces systèmes de l'art antérieur, l'ancrage du groupement électrochimiluminescent sur le support est réalisé par l'intermédiaire respectivement d'alcane bisphosphonate d'aluminium et d'aminohexane, couches non conductrices.

L'emploi d'un film conducteur électronique selon la présente invention favorise le transfert électronique support/luminol et donc les performances de ce mode de détection par ECL.

De plus, dans le procédé de la présente invention, le support présente une double fonction active permettant (i) l'immobilisation électro-contrôlée de l'objet biologique au sein du film conducteur électronique et (ii) l'activation en tant que "déclencheur" du processus de ECL du luminol ce qui permet son application à un système multiparamétrique (analyse en parallèle, ou "multi-array") de type biopuce basée sur la technologie développée depuis plusieurs années utilisant un réseau d'électrodes de type MICAM (marque déposée).

Chaque plot composant une biopuce de type MICAM reconnaissant de manière spécifique une molécule particulière, par exemple une séquence d'ADN, les différentes molécules, par exemple différentes séquences d'ADN, présentes dans un échantillon donné peuvent être détectée simultanément lors de l'analyse. Pour l'application de la présente invention, il suffit d'appliquer, soit de manière séquentielle, soit en parallèle, à chacun des plots d'une telle biopuce, le potentiel de déclenchement de l'ECL du marqueur, par exemple du luminol. Ainsi, par exemple pour de l'ADN, à partir des plots qui présentent une luminescence, il est possible d'identifier les séquences d'ADN présentes dans l'analyte. Le support présente une double fonction active permettant (i) l'immobilisation électro-contrôlée de l'objet biologique sonde, dans cet exemple l'ADN, et (ii) l'activation en tant que déclencheur de l'ECL.

La présente invention permet donc pour la première fois la mise en oeuvre d'un procédé d'électrochimiluminescence sur un système analyse en parallèle ("multi-array") dans lequel le support d'immobilisation est aussi celui qui permet le déclenchement de la chimiluminescence.

L'application de la présente invention à un système multiparamétrique permet en outre une lecture multiparamétrique et/ou en parallèle que n'offre pas le dispositif de positionnement successif décrit par le brevet Hashimoto et al. [6].

De plus, le procédé de la présente invention permet d'effectuer des analyses quantitatives directes tout en éliminant les problèmes de bruit de fond inhérents aux mesures par fluorescence.

Enfin, dans la présente invention, l'immobilisation du marqueur luminol est réalisée spécifiquement sur la molécule cible contrairement aux procédés de l'art antérieur faisant intervenir des intercalants électrochimiluminescent dans le cas de biocapteurs à ADN tel que décrit dans le document [6].

D'autres caractéristiques et avantages apparaîtront encore à l'homme du métier à la lecture des exemples qui suivent donnés à titre illustratif et non limitatif en référence aux figures annexées.

### BREVE DESCRIPTION DES FIGURES

Les figures 1 et 2 sont des représentations schématiques d'un transfert électronique direct (figure 1) et indirecte (figure 2) entre la molécule cible marquée et le support via le film conducteur électronique lors d'une lecture d'électrochimiluminescence selon la présente invention.

### EXEMPLES

### EXEMPLE 1 : ELECTROCHIMILUMINESCENCE DU LUMINOL EN SOLUTION SUR UNE ELECTRODE RECOUVERTE D'UN FILM DE POLYPYRROLE

Les inventeurs ont préalablement entrepris une série d'expériences d'électrochimiluminescence (ECL) afin de s'assurer qu'une émission lumineuse électrogénérée peut être activée à partir d'un support recouvert par un polymère conducteur électronique comme le polypyrrole, le groupement électroluminescent étant en solution et non immobilisé sur le film de polypyrrole.

L'étude de ECL est réalisée à partir d'une électrode ("Screen-printed") composée d'une électrode de travail en carbone (S = 0,2 cm²) et d'une électrode de référence AgCl/Ag. La mesure de luminescence est effectuée à l'aide d'un luminomètre qui mesure l'intensité luminescence émise à partir de l'électrode "Screen-printed" placée dans une cellule de mesure contenant 2 ml d'une solution de luminol de concentration donnée en milieu tampon Véronal-HCl 30 mM pH=9. La mesure de ECL est réalisée en appliquant tout d'abord un potentiel de +0,450 V entre l'électrode de carbone et l'électrode de référence puis en injectant au mélange précédent, 55 µl d'une solution d'eau oxygénée à 2 mM en milieu tampon Véronal. L'ensemble de ces opérations s'effectue sous agitation magnétique et en milieu protégé de la lumière.

Le film de polypyrrole est préalablement électrodéposé sur le support par voie électrochimique à potentiel imposé de +0,80 V/ECS sur l'électrode de carbone composant l'électrode "Screen-printed" par passage de 20 mC/cm² à partir d'une solution à 50 mM en monomère pyrrole dans H₂0/LiClO₄ 0,1 M. Cette électrosynthèse est réalisée dans une cellule d'électrochimie classique. Après son élaboration, le film est ensuite rincé par trempage dans une solution H₂0/LiClO₄ 0,1 M exempte de monomères. A l'issue de cette opération, l'électrode "Screen-printed" est placée dans la cellule de mesure en vue de réaliser la mesure de luminescence électrogénérée.

Dans ces conditions, il a été possible de détecter sur l'électrode de carbone recouverte d'un film de polypyrrole, une quantité minimale en luminol égale à 3 pmol (1,5 nM). Pour cette quantité, le rapport signal/bruit est inférieur à 3 ce qui indique que les inventeurs ont atteint la limite de détection.

En reproduisant cette mesure de ECL à partir d'une électrode de carbone nue, c'est à dire non recouverte par un film de polypyrrole, au contact d'une solution tampon Véronal contenant de 1,5 nM en luminol +55 µL d'une solution d'eau oxygénée à 2 mM, l'intensité lumineuse recueillie est alors 3 fois plus importante que celle mesurée précédemment.

Il apparaît donc que 2/3 de la luminescence est perdue lorsque l'électrode est recouverte d'un film de polypyrrole.

### EXEMPLE 2 : ELECTROCHIMILUMINESCENCE DU LUMINOL EN SOLUTION SUR UNE ELECTRODE RECOUVERTE D'UN FILM DE POLYPYRROLE SUROXYDE

Dans cet exemple, les inventeurs ont réalisé une mesure de luminescence à partir d'une électrode de carbone recouverte d'un film de polypyrrole suroxydé. Le dépôt du polypyrrole est effectué comme dans l'exemple 1.

La mesure de la luminescence issue de ECL du luminol en solution est réalisée selon le mode opératoire décrit dans l'exemple 1.

Toutefois, dans cet exemple, avant la mesure ECL et après l'électrosynthèse du film de polypyrrole, l'électrode de carbone recouverte du polypyrrole est portée pendant 5 minutes à un potentiel de 1 V/ECS en milieu H₂0/LiC1O₄ 0,1 M. Il a été montré dans la littérature que dans ces conditions le film de polypyrrole perdait ses propriétés de conduction électronique (le polypyrrole est dit suroxydé).

Les inventeurs ont constaté que sur cette électrode de carbone recouverte par un film de polypyrrole suroxydé et à partir d'une solution tampon Véronal contenant 3 nM de luminol + 55 µl d'une solution d'eau oxygénée à 2 mM, l'application d'une différence de potentiel de 0,450 V ne conduit pas à l'émission de luminescence. Dans ces conditions, si le film de polypyrrole est préalablement suroxydé, le phénomène d'ECL n'est alors plus détectable.

Il ressort de cette expérience que l'état conducteur électronique du film est important dans le processus ECL du luminol et que d'autre part, la chimiluminescence électrogénérée a lieu à l'interface film/solution.

A noter également qu'aucun exemple dans la littérature ne mentionne l'observation de luminescence par ECL induite par des unités électrochimiluminescentes, comme des complexes de ruthénium trisbipyridine, immobilisées dans un film de polypyrrole.

### EXEMPLE 3 : ECL DU LUMINOL IMMOBILISE SUR UN FILM DE POLYPYRROLE

Une première étude de ECL du luminol immobilisé sur un film de polypyrrole a été réalisée à partir d'un composé commercial dérivé du luminol, la streptavidine-isoluminol.

La streptatividine est une protéine sur laquelle sont greffées en moyenne trois molécules dérivées de l'isoluminol, l'ABEI (6-[N-(4-Aminobutyl)-N-éthyl]amino-2,3-dihydro- 1,4-phtalazine-1,4-dione). La streptavidine-isoluminol est ancrée sur un film de polypyrrole renfermant des entités biotine (polypyrrole biotine) via la forte interaction existante entre ces molécules de biotine et la streptavidine (constante d'affinité Ka = 10¹⁵).

Le film de polypyrrole biotine est électrosynthétisé sur l'électrode en carbone de l'électrode "Screen-printed" par balayages successifs en potentiel à 50 mV/s entre -0,3 V et +0,8 V vs Ag + 102 M/Ag à partir une solution 10 mM en monomère pyrrole biotine (A) dans CH₃CN/nBu₄PF₆ 0,1 M par passage de 20 mC/cm².

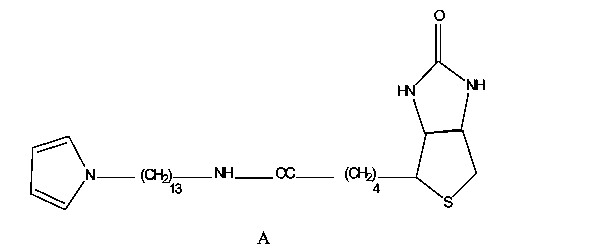

Après rinçage du film dans une solution tampon PBS pH = 7, ce film est mis en contact pendant 5 minutes avec une solution tampon PBS pH = 7 de streptavidine-isoluminol à 0,5 gl⁻¹. Ensuite, l'électrode est soigneusement rincée avec une solution tampon carbonate pH = 9,5 à 50 mM.

C'est à partir de cet assemblage moléculaire polypyrrole biotine/streptavidine-isoluminol qu'on réalise la mesure de ECL.

Comme dans les exemples précédents, l'électrode "Screen-printed" ainsi modifiée, est placée dans la cellule de mesure contenant 2 ml d'une solution tampon carbonate pH = 9,5 à 50 mM. Puis une différence de potentiel de +0,450 V est appliquée entre l'électrode de carbone et l'électrode AgCl/Ag avant l'injection dans la solution tampon carbonate, de 55 µl d'une solution d'eau oxygénée à 2 mM. On enregistre alors la variation de l'intensité lumineuse détectée par le photomètre.

Après addition de la solution d'eau oxygénée, une variation significative de l'intensité lumineuse est enregistrée correspondant à 120 u.a. (unité arbitraire) soit une quantité de streptavidine-isoluminol immobilisée d'environ 20 pmol.cm².

En reproduisant cette expérience à partir d'un film de polypyrrole ne renfermant pas d'unités biotine, nous avons pu estimer la quantité de streptavidine-isoluminol simplement adsorbée sur de tels polymères. Le film non biotinylé est électrosynthétisé à partir du monomère pyrrole noté (B):

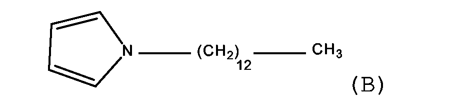

La mesure de l'intensité de ECL mesurée avec ce film non biotinylé est de 28 u.a. soit 5 pmol.cm⁻².

De ces deux mesures, on peut donc estimer que la quantité de streptavidine-isoluminol spécifiquement immobilisée sur le film de polypyrrole biotine est de 15 pmol.cm⁻².

Cette valeur est en accord avec celle obtenue par des mesures de microbalance à quartz.

### EXEMPLE 4 : DETECTION PAR ELECTROCHIMILUMINESCENCE DE LA RECONNAISSANCE ODN/ODN COMPLEMENTAIRE SUR UN FILM DE POLYPPYROLE BIOTINE

Dans cet exemple, les inventeurs démontrent que la reconnaissance entre un ODN sonde immobilisé et un ODN cible complémentaire peut être détectée via ECL par électrochimiluminescence.

La sonde ECL, ici la streptavidine-isoluminol, est couplée sur l'annalyte cible, à savoir l'ODN complémentaire. Quant à l'ODN sonde, il est immobilisé sur un film de polypyrrole biotine par l'intermédiaire de molécules d'avidine préalablement ancrées. Ainsi l'assemblage moléculaire mis en oeuvre dans cet exemple, correspond au système multicouches : polypyrrole biotine/avidine/ODN sonde-ODN cible/ streptavidine-isoluminol.

Tout d'abord, un film de polypyrrole biotine est électrosynthétisé sur le support selon les conditions opératoires décrites dans l'exemple 3. Après trempage dans une solution tampon PBS, ce film est ensuite abandonné pendant 5 minutes dans une solution d'avidine à 0,125 g.l⁻¹ en milieu PBS ce qui conduit à l'immobilisation de molécules d'avidine sur le film de polypyrrole biotine via l'interaction forte biotine/avidine. A l'issue, l'électrode est rincée à l'aide d'une solution de PBS puis mise en contact avec une solution ODN sonde biotinylée à 0,6 µM.

Du fait que chaque ODN sonde porte en son extrémité une fonction biotine, leur ancrage sur l'assemblage polypyrrole biotine/avidine se fait aisément par simple mise en contact permettant l'interaction entre les sites de l'avidine restés libres et les molécules biotine greffées sur les OND sondes.

La reconnaissance des ODN cibles par les ODN sondes immobilisés sur l'architecture polypyrrole biotine/avidine/ODN sonde. Comme précédemment, ceci est simplement réalisé par la mise en contact de cette architecture moléculaire avec une solution d'ODN complémentaire cible (ODN également biotinylé) à 0,6 µM en milieu PBS. Afin de s'assurer par ECL que l'hybridation entre les ODN sondes et les ODN cibles est bien effective, une molécule de streptavidine-isoluminol est ensuite greffée sur l'ODN cible apparié à l'ODN sonde. Là aussi, ce greffage est simplement réalisé par simple interaction biotine/avidine en sachant que l'ODN cible porte en son extrémité une unité biotine.

Avant d'entreprendre la mesure ECL proprement dite, l'ensemble polypyrrole biotine/avidine/ODN sonde - ODN cible/streptavidine-isoluminol ancré sur l'électrode de carbone d'une électrode "Screen-printed", est soigneusement rincée avec une solution tampon carbonate pH = 9,5 à 50 mM. Puis, comme précédemment, la mesure de ECL est effectuée en plaçant l'électrode "Screen-printed" dans la cellule de mesure contenant 2 ml de la solution tampon carbonate pH = 9,5. Après injection à ce milieu de 55 µl d'une solution d'eau oxygénée à 2 mM, on applique une différence de potentiel de 0,450 V entre l'électrode de carbone et l'électrode de référence AgCl_{(S)}/Ag. La mesure de l'intensité lumineuse émise par ECL est en moyenne de 65 u.a. ce qui correspond à une immobilisation surfacique de streptavidine-isoluminol égale à 10 pmol cm⁻². De ce résultat, il ressort que la détection et la quantification d'un événement biologique telle que l'hybridation entre un ODN sonde et un ODN complémentaire cible ayant lieu à l'interface d'un support conducteur électrique est réalisable par ECL.

Afin de s'assurer que l'électrochimilunescence observée est bien due à l'hybridation entre les oligonucléotides ODN sondes/ODN cibles, les inventeurs ont réalisé une expérience similaire où cette fois-ci, l'assemblage polypyrrole biotine/avidine/ODN cible est mis en contact avec une solution contenant non pas des ODN complémentaires cibles mais simplement des ODN non-complémentaires. Dans ce cas, aucune reconnaissance spécifique par hybridation ne se produit entre les ODN sondes immobilisées et les ODN non-complémentaires en solution.

Par suite, la streptavidine-isoluminol ne peut alors s'immobiliser sur le système polypyrrole biotine/avidine/ODN cible faute de points d'ancrage biotine. C'est pourquoi comme attendu, aucune luminescence n'a été détectée dans ce cas après injection de 55 µL de la solution d'eau oxygénée à 2 mM et l'application du potentiel, confirmant ainsi que celle observée précédemment était effectivement le résultat de l'hybridation entre l'ODN sonde et l'ODN complémentaire cible.

### EXEMPLE 5 : REPRODUCTIBILITE DE LA DETECTION PAR ECL DE LA RECONNAISSANCE ODN/ODN COMPLEMENTAIRE SUR UN FILM DE POLYPYRROLE BIOTINE

Comme montré dans le document [7], il est possible à l'aide d'un détergent puissant, le dodécylesulfate de sodium (SDS), de détruire les interactions biotine/avidine ce qui induit la régénération de la matrice polymère polypyrrole-biotine.

Les inventeurs ont donc étudié la possibilité de pouvoir réutiliser une deuxième fois l'assemblage polypyrrole biotine/avidine/ODN sondes - ODN complémentaires cibles/streptavidine-isoluminol, pour une autre mesure ECL.

C'est pourquoi après la première mesure de ECL réalisée sur l'assemblage polypyrrole biotine/avidine/ODN sondes - ODN complémentaires cibles/streptavidine-isoluminol, cet ensemble a été traité par une solution aqueuse de SDS à 50 mM et 60°C afin de détruire cette architecture moléculaire et de pouvoir retrouver ancré sur l'électrode de carbone que le film polypyrrole biotine.

Après un rinçage soigneux à l'eau permutée, un nouvel assemblage identique au premier (polypyrrole biotine/avidine/ODN sondes -ODN complémentaires cibles/streptavidine-isoluminol) a été réalisé sur ce film de polypyrrole biotine régénéré.

Une fois ce nouvel assemblage réalisé, le luminophore est excité comme précédemment sous un potentiel de 0,450 V en plongeant l'électrode dans un milieu composé de 2 ml de solution tampon carbonate à 50 mM pH = 9,50, 55 µl de la solution d'eau oxygénée à 2 nM. L'évolution de l'intensité lumineuse enregistrée au cours de cet expérience donne une variation de 55 u. a. Cette valeur est légèrement inférieure à celle obtenue lors de la première mesure et correspond à une concentration en streptavidine-isoluminol de 8 pmol cm².

Cette étude montre la possibilité de détecter une nouvelle fois l'hybridation de l'ADN après régénération de la matrice polypyrrole biotine.

### REFERENCES BIBLIOGRAPHIQUES

[1] Fahnrich K. A., Pravda M. and Guibault G. G.,"Recent applications of electrogenerated chemiluescence in chemical analysis" *Talanta 2001*, **54**(4), 531-559.
[2] US Patent WO 9639534 "Electrochemiluminescence enzyme biosensors" Martin M. T.
[3] Blackurn G. F., Shah H. P., Kenten J. H., Leland J., Kamin R. A., Link J., Peterman J., Powell M. J., Shah A., Talley D. B., Tyagi S. K., Wilkins E., Wu T. G. and Massey R. J., *Clin. Chem 1991*, **37,** 1534.
[4] US Patent WO 9606946 "Biosensor for and method of electrogenerated chemiluminescent detection of nucleic acid adsorbed to a solid surface" Bard A. J. and Xu X-H.
[5] Marquette C. A.. and Blum L. J., "Electrochemiluminescence of luminol for 2,4-D optical immunosensing in a flow injection analysis system", *Sensors Actuat. B* 1998, **51**, 100-106.
[6] US Patent 5,776,672 7 juillet 1998, "gene detection method", K. Hashimoto, K. Ito, Y. Ishimori, and M. Gotoh.
[7] A. Dupont-Filliard, A, Roget, T. Livache et M. Billon, "Reversible oligonucleotide immobilization based on biotinylated polypyrrole film", *Anal. Chem. Acta* 2001, **449**, 45-50.
[8] R.J. Foster end C.F. Hogan, Anal. Chem. 2000 (72) 5576.
[9] *Analytical Chemistry* Vol. 48, n°13, Novembre 1976, pp.1933.
[10] *Analytical Biochemistry,* 111, 87-96 (1981) p.87.

## Revendications

1. Procédé de détection d'une reconnaissance moléculaire entre une molécule sonde fixée sur un support et une molécule cible recherchée dans un échantillon à tester, ledit procédé comprenant les étapes suivantes :
a) déposer sur un support un film conducteur à conduction électronique ou rédox et fixer sur ledit film une molécule sonde pour obtenir un support test,
b) marquer avant ou après l'étape c) la molécule cible avec un marqueur électrochimiluminescent,
c) mettre en contact la molécule cible avec le support test dans des conditions physiques et chimiques permettant la reconnaissance moléculaire et l'assemblage spécifique entre la molécule sonde et la molécule cible,
d) rincer le support test de manière à supprimer l'excès de marqueur électrochimiluminescent tout en préservant l'assemblage spécifique entre la molécule sonde et la molécule cible de l'étape c), et
e) soumettre le support test rincé obtenu à l'étape d) à une lecture du signal d'électrochimiluminescence déclenchée par un transfert électronique direct, ou indirect, entre la cible et le support via le film conducteur électronique.

2. Procédé selon la revendication 1, dans lequel l'étape a) est une électro-copolymérisation, sur le support, de monomères précurseurs du polymère conducteur avec des monomères précurseurs fonctionnalisés par la molécule sonde.

3. Procédé selon la revendication 1, dans lequel le film conducteur électronique est un film de polypyrrole.

4. Procédé selon la revendication 1, dans lequel la molécule sonde est fixée au film conducteur par un système avidine/biotine.

5. Procédé selon la revendication 1, dans lequel la molécule sonde est de l'ADN, un anticorps, une protéine ou un enzyme.

6. Procédé selon la revendication 1, dans lequel le marqueur électrochimiluminescent est choisi dans le groupe constitué du luminol, de l'isoluminol, d'un aminophtalhydrazine et de leurs dérivés.

7. Procédé selon la revendication 1, dans lequel le marqueur électrochimiluminescent est choisi dans le groupe constitué des dérivés du luminol ou de l'isoluminol de formules suivantes : 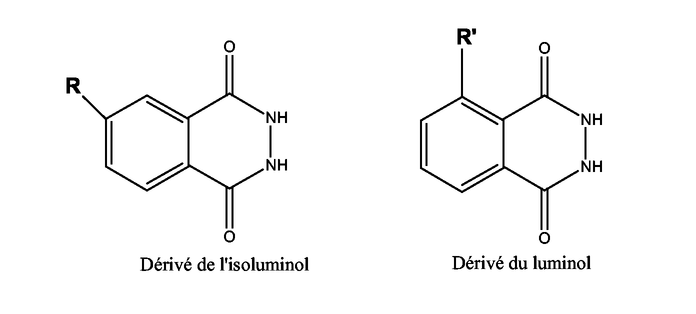 dans lesquelles R ou R' sont des groupements actifs qui permettent le marquage de la molécule cible choisis parmi H₂N-(H₂C)₃-NH ; NH₂-(CH₂)₄-(C₂H₅)N ou des dérivés de ceux-ci.

8. Procédé selon la revendication 1, dans lequel, dans l'étape e), le transfert électronique entre la cible et le support via le film conducteur électronique étant indirect, il est réalisé au moyen d'un médiateur rédox choisi parmi un groupement métallocène tel que le ferrocène ou le diferrocène, des complexes de métaux de transition tels qu'un complexe de cobalt, en solution ou greffé au film conducteur ou à la molécule sonde, ou des intercalants de la molécule sonde possédant des propriétés rédox ou comportant des groupements rédox.

9. Utilisation d'un procédé selon l'une quelconque des revendications 1 à 9, pour une analyse quantitative ou qualitative d'une molécule cible présente dans un échantillon.

10. Utilisation d'un procédé selon l'une quelconque des revendications 1 à 9, dans un système d'analyse multiparamétrique et/ou en parallèle.
